# EUROPEAN PATENT SPECIFICATION

(11) **EP 2 262 486 B1**
(45) Date of publication and mention of the grant of the patent: **02.01.2013**
(21) Application number: 09777586.0
(22) Date of filing: 31.07.2009
(51) Int. Cl.: A61K 9/20, A61K 31/554

(54) **QUETIAPINE COMPOSITION**
QUETIAPIN-ZUSAMMENSETZUNG
COMPOSITION DE QUÉTIAPINE

(30) Priority: 01.08.2008 SI 200800193; 18.03.2009 SI 200900073
(43) Date of publication of application: 22.12.2010
(73) Proprietor: KRKA, Tovarna Zdravil, D.D., Novo Mesto, 8501 Novo Mesto (SI)
(72) Inventor: PAVLI, Matej, SL-1000 Ljubljana (SI); DREU, Rok, 2380 Slovenj Gradec (SI); BAUMGARTNER, Sasa, SL-1000 Ljubljana (SI); PLANINSEK, Odon, SL-1000 Ljubljana (SI); PISEK, Robert, SL-1000 Ljubljana (SI); VRECER, Franc, SL-8351 Straza pri Novem mestu (SI); VRBINC, Miha, SL-8000 Novo Mesto (SI)
(74) Representative: UEXKÜLL & STOLBERG
(86) International application number: PCT/EP2009/005573
(87) International publication number: WO 2010/012490

(56) References cited:
- EP-A1- 0 240 228
- WO-A1-97/45124
- WO-A1-03/039516
- US-A1- 2005 158 383
- GUPTA V K; HARIHARAN M; WHEATLEY T A; PRICE J C: "Controlled-release tablets from carrageenans: effect of formulation, storage and dissolution factors" EUROPEAN JOURNAL OF PHARMACEUTICS AND BIOPHARMACEUTICS, ELSEVIER SCIENCE PUBLISHERS B.V., AMSTERDAM, NL, vol. 51, no. 3, 1 May 2001 (2001-05-01), pages 241-248, XP004239485 ISSN: 0939-6411

## Description

### FIELD OF THE INVENTION

The present invention relates to novel sustained-release pharmaceutical composition comprising quetiapine and Carrageenan in the form of a tablet.

### BACKGROUND OF THE INVENTION

Quetiapine is an atypical antipsychotic with established efficacy in the treatment of schizophrenia, shows efficacy in the treatment of acute mania and depression associated with bipolar disorder. Quetiapine, either as monotherapy or in combination with lithium or divalproex sodium (valproate semisodium), is generally well tolerated and effective in reducing manic symptoms in adult and adolescent patients with acute bipolar mania, and is approved for use in adults for this indication. As monotherapy, the drug is also effective in reducing depressive symptoms in patients with bipolar depression.

Quetiapine and its hemifumarate salt was first disclosed in EP240228. Later were published many more patent applications concerning quetiapine such as EP282236, EP907364, WO9906381, EP1218009, EP1448169, EP1482945, WO2005028457, WO2005041935, WO2007036599, EP1252151.

Carrageenan is the hydrocolloid obtained by extraction with water or aqueous alkali,from some members of the class *Rhodophyceae* (red seaweeds). Carrageenan consists chiefly of potassium, sodium, calcium, magnesium, and ammonium sulfate esters of galactose and 3,6-anhydrogalactose copolymers. These hexoses are alternately linked a-1,3 and b-1,4 in the polymer. The prevalent copolymers in the hydrocolloid are designated *kappa-, iota-,*and *lambda*-carrageenan. *Kappa*-carrageenan is mostly the alternating polymer of D-galactose-4-sulfate and 3,6-anhydro-D-galactose. *Iota*-carrageenan is similar, except that the 3,6-anhydrogalactose is sulfated at carbon 2. Between *kappa*-carrageenan and *iota*-carrageenan there is a continuum of intermediate compositions differing in degree of sulfation at carbon 2. In *lambda*-carrageenan, the alternating monomeric units are mostly D-galactose-2-sulfate (1,3-linked) and D-galactose-2,6-disulfate (1,4-linked). The ester sulfate content for Carrageenan ranges from 18 percent to 40 percent. In addition, it contains inorganic salts that originate from the seaweed and from the process of recovery from the extract. λ-Carrageenan (lambda-carrageenan) is a non-gelling polymer containing about 35% ester sulfate by weight and no 3,6-anhydrogalactose.

WO 97/45124 describes a sustained-release formulation of quetiapine comprising hydrophilic matrix comprising a gelling agent. The drawback of such a composition is that the gelling level of the composition and thus also its dissolution rate is difficult to control as it also depends on physiological factors.

WO 03/000293 relates to an oral pharmaceutical formulation comprising iota-carageenan, one or more gelling polymers, and a basic pharmaceutically active ingredient. Pharmaceutical formulations comprising quetiapine are not described.

WO 03/039516 discloses a method of improving dissolution of a poorly dispersible medicament e.g. quetiapine fumarate, which comprises mixing the poorly dispersible medicament with a floating agent and granulating the mixture. As floating agents non-water soluble cellulose, sodium alginate, propylene glycol alginate, tragacanth powder or xanthan gum are mentioned.

### BRIEF DESCRIPTION OF THE DRAWINGS

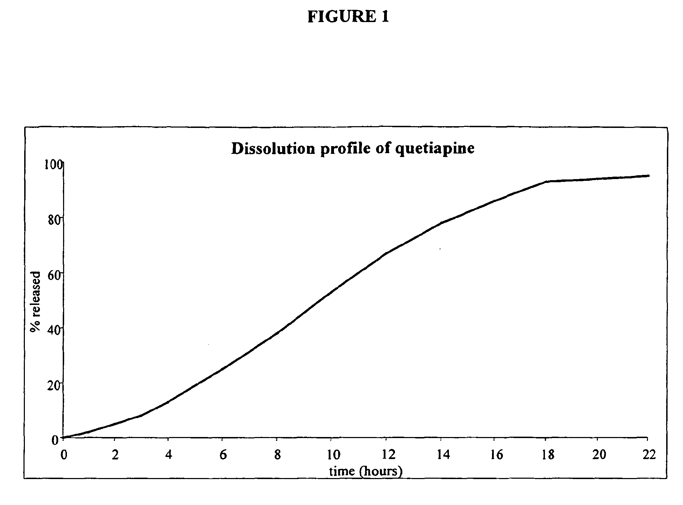

Figure 1 is a dissolution profile of quetiapine from tablets according to example 22.

### DETAILED DESCRIPTION OF THE INVENTION

The problem solved by the present invention is the provision of a pharmaceutical composition overcoming the above mentioned drawbacks. (i.e., difficult control of dissolution rate due to the gelling agent present in the formulation).

The present invention describes a pharmaceutical composition in the form of a tablet and processes for its preparation comprising solid matrix system of quetiapine or its salt and a non-swelling (*i.*e. non-gelling) agent such as non-gelling carrageenan. The composition according to present invention is capable of prolonging the release of quetiapine from the composition to at least 12 hours, preferably to at least 16 hours and most preferably to at least 20 hours.

Preferred embodiments of the invention are described in the dependent claims.

The pharmaceutical composition of present invention can be produced by manufacturing processes known from the state of the art such as direct compression of a powder mixture of active substance and selected excipients or compression of a pregranulated powder mixture of active substance and selected excipients. Granulation can be performed by dry processes, wet or hot melt processes. Dry granulation can be performed by state of the art processes such as slugging or compaction, where a powder mixture of drug and selected excipients is compressed into comprimates by using equipment such as a slugging machine or roller compactor followed by crushing the obtained comprimates into granulate and optionally sieving of the granulate. Wet granulation processes can be performed by using granulators such as a low shear or high shear granulator or fluid bed granulator, where agglomeration of particles is obtained by addition of granulation liquid containing a solvent selected from water, organic solvents such as alcohols with one to four carbon atoms or ketones such as acetone or esters such as ethyl acetate or the mixture thereof in which optionally excipients selected from diluents, binders, antioxidants, surfactants, organic acids can be dissolved, suspended or emulsified. Hot melt granulation can be performed by state of the art processes such as hot melt extrusion or melt granulation. Hot melt extrusion is performed by extrusion of the heated mixture of extrusion polymer, active substance and optionally other excipients selected from carrageenan, anionic polymer, organic acid, diluent or lubricant, followed by cooling, milling and optionally sieving the obtained extrudate to obtain a granulate of desired particle size distribution. Melt granulation can be performed by agglomeration of a powder mixture of active substance and excipients selected from carrageenan, anionic polymer, organic acid and optionally other excipients selected from diluents antioxidants or surfactants with a molten binder having a melting point of below 130°C, preferably below 100°C and most preferably below 80°C by the state of the art processes such as melt granulation in high shear or fluid bed granulators.

Preferably, carrageenan is lambda-carrageenan and quetiapine is preferably in the form of quetiapine hemifumarate. More preferably the quetiapine hemifumarate is quetiapine hemifumarate of form I as defined in WO 03/080065.

The active ingredient is preferably used in the form of powder having an average particle size of 5 to 300 µm.

The compositions of the present invention preferably comprise 10 to 80 wt.-%, more preferably 25 to 55 wt-% and most preferably 30 to 45 wt-% of quetiapine. For example the compositions can contain 50, 150, 200, 300, 400, 600 or 800 mg of quetiapine. Particularly preferred are compositions which contain 200 or 400 mg of quetiapine, preferably in the form of quetiapine hemifumarate.

The compositions of the present invention preferably comprise 5 to 80 wt.-%, preferably 15 to 65 wt.-% and most preferably 20 to 55 wt.-% of non-gelling carrageenan, preferably lambda-carrageenan.

If not stated otherwise all percentages herein are by weight (% w/w) and are based on the total weight of the composition.

The pharmaceutical compositions according to the present invention an present in the form of tablets which can be manufactured by direct compression or granulation. Water, organic solvents miscible with water such as ethanol, isopropanol, methanol, acetone or the mixture thereof can be used as granulation liquid.

Additional excipients can be used in the composition selected from diluents, binders, drug release rate modifiers, disintegrants, glidants or lubricants.

Diluents can be selected from water soluble diluents such as lactose, mannitol, dextran or from water insoluble diluents such as crystalline cellulose such as microcrystalline cellulose, earth-alkali salts of phosphoric acid such as calcium hydrogen phosphate in anhydrous or hydrated state etc. Diluents are preferably used in the range from 5 to 65 wt.-%, more preferably in the range from 10 to 40 wt.-% and most preferably 20 to 27 wt.-%. Lactose, in particular alpha-lactose monohydrate, and mannitol are preferred. Alpha-lactose monohydrate is commercially available under the trademark Tablettose®.

Binders can be selected from non-gelling binders such as low MW povidone, for example Povidone K12, K17, K25 and/or K30. They are preferably used in the range from 1 to 5%. Surfactants can be selected from anionic and or non-ionic surfactants such as sodium dodecylsulphate sulphate.

Lubricants can be selected from metal stearates, sodium starch fumarate, talc, sodium benzoate or mixtures thereof.

One aspect of the present invention is a pharmaceutical composition comprising quetiapine or its salt, non-gelling carrageenan and an organic acid. Preferably, the carrageenan is lambda-carrageenan and quetiapine is preferably in the form of quetiapine hemifumarate. The organic acid can be selected from the group consisting of citric acid, fumaric acid, tartaric acid, benzoic acid, malic acid, ascorbic acid, succinic acid and/or mixtures thereof. Salts of these acids can also be used. Acids are preferably used in an amount of 1 to 30 wt.-%, more preferably 5 to 15 wt.-%.

Another aspect of present invention is a pharmaceutical composition comprising quetiapine or its salt, non-gelling carrageenan and an anionic polymer. Preferably, the carrageenan is lambda-carrageenan and quetiapine is preferably in the form of quetiapine hemifumarate. The anionic polymers can be selected from the group comprising cellulose acetate phathalate such as Eastman C-A-P Cellulose Ester; methacrylic acid copolymer such as Eudragit L, Eudragit S and Eudragit FS types; cellulose acetate succinate such as Hypromellose (HPMC) Acetate Succinate (HPMCAS, Aqoat®); polyvinyl acetate phthalate such as Sureteric; hydroxypropylmethyl cellulose phthalate (HPMCP) such as HPMCP-50, HPMCP-55 and/or mixtures thereof. The preferred anionic polymers are HPMC acetate succinate polymers, such as the commercially available Aquoat (AS-HF, AS-MF, AS-LF, AS-HG, AS-MG, AS-LG), methacrylic acid - ethyl acrylate copolymers, such as the commercially available Eudragit L100-55 and methacrylic acid - methyl methacrylate copolymer such as the commercially available Eudragit L100. The one or more anionic polymers are preferably used in an amount of 1 to 90 wt.-%, more preferably 5 to 60wt.-% and most preferably 10 to 35 wt.-%.

A still further aspect of present invention is a pharmaceutical composition comprising quetiapine or its salt, non-gelling carrageenan and PEG (polyethylene glycol). Preferably, the carrageenan is lambda-carrageenan and quetiapine is preferably in the form of quetiapine hemifumarate. PEG is preferably of low MW (molecular weight) PEG, more preferably PEG having an average MW within the range of from 4,000 to 8,000 g/mol such as PEG 4000 or PEG 8000. The polyethylene glycol is preferably used in an amount of 4 to 50 wt.-%, more preferably 8 to 35 wt.-% and most preferably 12 to 24 wt.-%.

Another aspect of present invention is a pharmaceutical composition comprising quetiapine or its salt, non-gelling carrageenan and mannitol. Preferably, the carrageenan is lambda-carrageenan and quetiapine is preferably in the form of quetiapine hemifumarate.

The organic acid, anionic polymer, PEG and/or mannitol can be optionally used together.

According to a particularly preferred embodiment the pharmaceutical compositions of the present invention comprise:
25 to 55 wt.-%, preferably 30 to 45 wt.-% of quetiapine, preferably in the form of quetiapine hemifumarate,
15 to 65 wt.%, preferably 20 to 55 wt-% of non-gelling carrageenan, preferably lambda carrageenan,
and optionally 0,5 to 2 wt.-% of lubricant, preferably magnesium stearate.

According to another preferred embodiment the pharmaceutical compositions of the present invention additionally comprise:
10 to 40 wt.-% diluent, preferably, lactose, in particular alpha-lactose monohydrate, mannitol and/or dicalcium phosphate dihydrate, and/or
5 to 15 wt.-% organic acid, preferably citric acid and/or fumaric acid, and/or
10 to 35 wt.-% anionic polymer, in particular HPMC acetate succinate and/or methacrylic acid copolymer, and/or
12 to 24 wt.-% polyethylene glycol, in particular polyethylene glycol having an average molecular weight of 4,000.

If the pharmaceutical composition of present invention is present in the form ot a tablet, the tablet can optionally be film-coated which improves the appearance and physical-chemical characteristics of the tablet without influence on the release profile of quetiapine. For example suitable coating agents can be selected from the group consisting of polyvinylalcohol (PVA), copovidone based coatings (Kollidon VA-64), HPMC based coatings (Opadry®), sodium carboxymethylcellulose, coatings based on block copolymer of polyvinyl alcohol and polyethylenglycol and/or methacrylate copolymers (Eudragit EPO). Such coatings can optionally contain further ingredients such as stabilizers, antitacking agents, plasticizers, pigments and colorants.

Lambda carrageenan can have average particle size in the range of 5 to 500 µm. The organic acid can have average particle size in the range of 5 to 1000 µm.

The pharmaceutical composition of present invention can be in the form of round, capsule like or oval tablets with a maximal diameter in the range of 8 to 16 mm, preferably 10-14 mm. Crushing strength of the uncoated matrix tablets can be between 80 to 200 N, preferably 100 to 160N.

The pharmaceutical composition according to present invention can be packed into appropriate primary packaging such as high density polyethylene (HDPE) containers with closure such as polypropylene (PP) closure and with or without desiccator, aluminium foil blisters or polychlorotrifluoroethylene (Aclar®) blisters or any other suitable water vapour low permeable packaging such as blisters made of multilayer polymeric foil where different polymeric materials are combined into single foil.

Additionally inert gases such as nitrogen, argon or helium or vacuum can be used to assure inert atmosphere around the dosage form, such as tablet or capsule, in the sealed primary packaging. Inert atmosphere according to present invention mean that concentration of oxygen in the atmosphere around the solid dosage form such as tablet containing quetiapine or its salt packed in the primary packaging is less than 10 vol%, preferably less than 5 vol% and most preferably less than 2 vol%. The concentration of oxygen in the atmosphere surrounding the tablet can be determined by gas chromatography.

The invention is illustrated by reference to the following examples. However, the examples are not intended to limit the scope of the claims in any way. It will be apparent to those skilled in the art that many modifications, both to materials and methods, may be practiced without departing from the purpose and interest of this invention.

### EXAMPLES

Dissolution tests as shown in Figure 1 were performed using USP apparatus 1, baskets at 100 rpm, 900 ml of phosphate buffer pH 6.8. The HPLC evaluation of dissolution samples was performed with Gemini C-18, 50mm x 4,6mm, 5µm analytical column. Chromatographic conditions used were: detection at 250 nm, mobile phase buffer pH 9.0 (3,08g CH₃COONH₄ /1000ml H₂O, pH adjusted to 9.0 with triethylamine C₆H₁₅N) : CH₃CN = 50:50 (V/V), flow 1,0 ml/min, injection volume 10 µL, column temperature 30°C.

### Quetiapine hemifumarate particle size

Table 1 depicts particle size distribution of batches of quetiapine hemifumarate used in the preparation of formulations according to present invention. Quetiapine hemifumarate was used in a crystalline form I according to WO03/080065.

**Table 1**

| Batch | 1 | 2 | 3 | 4 | 5 | 6 | 7 | 8 |
|---|---|---|---|---|---|---|---|---|
| D[4,3] [µm] | 38 | 73 | 55 | 163 | 81 | 34 | 11 | 25 |
| d10 [µm] | 6.6 | 13.4 | 6.4 | 11.1 | 13.1 | 3.0 | 3.0 | 2.8 |
| d50 [µm] | 29.7 | 39.3 | 39.6 | 152.6 | 43.9 | 23.2 | 10.3 | 15.8 |
| d90 [µm] | 82.9 | 153.8 | 125.9 | 316.4 | 212.9 | 78.8 | 20.8 | 59.8 |

| | | | | | | | | |
|---|---|---|---|---|---|---|---|---|
| D[4,3]... volume mean diameter of particles (average particle diameter). D10... at least 10% of the particles have volume diameter of less than the specified value. D50... at least 50% of the particles have volume diameter of less than the specified value. D90... at least 90% of the particles have volume diameter of less than the specified value. | | | | | | | | |

The specific surface area of the samples was between 0.3 and 5 sq.m/g using 6 point BET method.

The average particle diameter was determined by laser light scattering method using a Malvem-Mastersizer Apparatus MS 2000 with Isopar L as dilution medium. Volume particle size distribution is determined by measuring the angular distribution of laser light scattered by a homogeneous suspension of particles.

### Fumaric acid particle size

Table 2 depicts particle size distribution of fumaric acid used in the examples of the present invention.

**Table 2**

| Batch | 1 | 2 |
|---|---|---|
| D[4,3] [µm] | 186 | 13 |
| d10 [µm] | 27,7 | 2,4 |
| d50 [µm] | 164,4 | 8,6 |
| d90 [µm] | 374,8 | 27,9 |

### Trade name definitions

| Trade name | Generic description | Supplier |
|---|---|---|
| Eudragit L100-55 | Methacrylic Acid - Ethyl Acrylate Copolymer (1:1), powder | Evonik |
| Eudragit L100 | Methacrylic Acid - Methyl Methacrylate Copolymer (1:1)" | Evonik |
| Aqoat AS - HF | Hydroxypropyl methylcellulose acetate succinate; acetyl content 10-14%; succinoyl content 4-8%; micronized | Shin-Etsu |
| Tablettose | Aglomerated lactose | Meggle |
| Povidone K30 | Polyvinylpyrrolidone with average molecular weight (weight-average) of 44000-54000 g/mol | Basf |

### PROCEDURE FOR EXAMPLES 1-25:

### a) DIRECT COMPRESSION:

Excipients are mixed. Active ingredient is added and mixed with excipients. To the obtained mixture magnesium stearate is added, homogenous mixture is finally mixed and compressed on tabletting machine.
Or

### b) WET GRANULATION:

Excipients except magnesium stearate are mixed. Active ingredient is added and mixed with excipients and granulated with granulation liquid. To the obtained granulate magnesium stearate is added, homogenous mixture is finally mixed and compressed on tabletting machine.

### Example 1

| Ingredient | mg/tablet core |
|---|---|
| Quetiapine hemifumarate | 400.0* |
| Lambda-carrageenan | 481.1 |
| Magnesium stearate | 8.9 |

| | |
|---|---|
| *...mass of quetiapine free base | |

### Example 2

| Ingredient | mg/tablet core |
|---|---|
| Quetiapine hemifumarate | 400.0* |
| Lambda-carrageenan | 240.55 |
| Dicalcum phosphate dihydrate (CaHPO4.2H2O Bekapress) | 240.55 |
| Magnesium stearate | 8.9 |

| | |
|---|---|
| *...mass of quetiapine free base | |

### Example 3

| Ingredient | mg/tablet core |
|---|---|
| Quetiapine hemifumarate | 300.0* |
| Lambda-carrageenan | 321.4 |
| Sodium dodecyl sulphate | 33.02 |
| Magnesium stearate | 13.36 |

| | |
|---|---|
| *...mass of quetiapine free base | |

### Example 4

| Ingredient | mg/tablet core |
|---|---|
| Quetiapine hemifumarate | 300.0* |
| Lambda-carrageenan | 177.21 |
| Lactose | 177.21 |
| Magnesium stearate | 13.36 |

| | |
|---|---|
| *...mass of quetiapine free base | |

### Example 5

| Ingredient | mg/tablet core |
|---|---|
| Quetiapine hemifumarate | 230.3 |
| Lambda-carrageenan | 154.6 |
| Tablettose | 154.6 |
| Citric acid | 61.3 |
| Magnesium stearate | 12.26 |

### Example 6

| Ingredient | mg/tablet core |
|---|---|
| Quetiapine hemifumarate | 230.3 |
| Lambda-carrageenan | 309.2 |
| Citric acid | 61.3 |
| Magnesium stearate | 12.26 |

### Example 7

| Ingredient | mg/tablet core |
|---|---|
| Quetiapine hemifumarate | 230.3 |
| Lambda-carrageenan | 185.2 |
| Aqoat AS - HF | 185.2 |
| Magnesium stearate | 12.26 |

### Example 8

| Ingredient | mg/tablet core |
|---|---|
| Quetiapine hemifumarate | 230.3 |
| Lambda-carrageenan | 185.2 |
| Eudragit L100-55 | 185.2 |
| Magnesium stearate | 12.26 |

### Example 9

| Ingredient | mg/tablet core |
|---|---|
| Quetiapine hemifumarate | 230.3 |
| Lambda-carrageenan | 339.8 |
| Citric acid | 30.65 |
| Magnesium stearate | 12.26 |

### Example 10

| Ingredient | mg/tablet core |
|---|---|
| Quetiapine hemifumarate | 230.3 |
| Lambda-carrageenan | 154.6 |
| Aqoat AS - HF | 154.6 |
| Citric acid | 61.3 |
| Magnesium stearate | 12.26 |

### Example 11

| Ingredient | mg/tablet core |
|---|---|
| Quetiapine hemifumarate | 230.3 |
| Lambda-carrageenan | 103.1 |
| Aqoat AS - HF | 206.1 |
| Citric acid | 61.3 |
| Magnesium stearate | 12.26 |

### Example 12

| ingredient | mg/tablet core |
|---|---|
| Quetiapine hemifumarate | 230.3 |
| Lambda-carrageenan | 185.5 |
| Eudragit L100-55 | 123.7 |
| Citric acid | 61.3 |
| Magnesium stearate | 12.26 |

### Example 13

| Ingredient | mg/tablet core |
|---|---|
| Quetiapine hemifumarate | 230.3 |
| Lambda-carrageenan | 309.2 |
| Fumaric acid | 61.3 |
| Magnesium stearate | 12.26 |

### Example 14

| Ingredient | mg/tablet core |
|---|---|
| Quetiapine hemifumarate | 230.3 |
| Lambda-carrageenan | 309.2 |
| Fumaric acid | 40.9 |
| Fumaric acid disodium salt anhydrous | 20.4 |
| Magnesium stearate | 12.26 |

### Example 15

| Ingredient | mg/tablet core |
|---|---|
| Quetiapine hemifumarate | 230.3 |
| Lambda-carrageenan | 259.3 |
| PEG 4000 | 111.1 |
| Magnesium stearate | 12.26 |

### Example 16

| Ingredient | mg/tablet core |
|---|---|
| Quetiapine hemifumarate | 230.3 |
| Lambda-carrageenan | 247.9 |
| Mannitol | 122.6 |
| Magnesium stearate | 12.26 |

### Example 17

| Ingredient | mg/tablet core |
|---|---|
| Quetiapine hemifumarate | 230.3 |
| Lambda-carrageenan | 185.2 |
| Eudragit L 100 - 55 | 185.2 |
| Magnesium stearate | 12.26 |

### Example 18

| Ingredient | mg/tablet core |
|---|---|
| Quetiapine hemifumarate | 230.3 |
| Lambda-carrageenan | 123.9 |
| Eudragit L 100 - 55 | 123.9 |
| PEG 4000 | 122.6 |
| Magnesium stearate | 12.26 |

### Example 19

| Ingredient | mg/tablet core |
|---|---|
| Quetiapine hemifumarate | 230.3 |
| Lambda-carrageenan | 123.9 |
| Eudragit L 100 - 55 | 123.9 |
| Mannitol | 122.6 |
| Magnesium stearate | 12.26 |

### Example 20

| Ingridient | mg/tablet core |
|---|---|
| Quetiapine hemifumarate | 230.3 |
| Carrageenan Lambda | 181.6 |
| PEG 4000 | 77.82 |
| Fumaric acid | 84.36 |
| Povidone K30 | 22.96 |
| Mg stearate | 3.00 |

### Example 21

| Ingridient | mg/tablet core |
|---|---|
| Quetiapine hemifumarate | 230.3 |
| Carrageenan Lambda | 181.6 |
| PEG 4000 | 77.82 |
| Fumaric acid | 42.38 |
| Povidone K30 | 22.96 |
| Mg stearate | 2.80 |

### Example 22

| Ingridient | mg/tablet core |
|---|---|
| Quetiapine hemifumarate | 230.3 |
| Carrageenan Lambda | 216.7 |
| PEG 4000 | 92.0 |
| Fumaric acid | 47.0 |
| Povidone K30 | 28.0 |
| Mg stearate | 6.00 |

### Example 23

| Ingridient | mg/tablet core |
|---|---|
| Quetiapine hemifumarate | 230.3 |
| Carrageenan Lambda | 62.24 |
| Eudragit L 100-55 | 124.5 |
| Mannitol | 180.0 |
| Mg stearate | 3.00 |

### Example 24

| Ingridient | mg/tablet core |
|---|---|
| Quetiapine hemifumarate | 230.3 |
| Carrageenan Lambda | 144.5 |
| Eudragit L 100-55 | 72.24 |
| PEG 4000 | 92.0 |
| Fumaric acid | 47.0 |
| Povidone K30 | 28.0 |
| Mg stearate | 6.00 |

### Example 25

| Ingridient | mg/tablet core |
|---|---|
| Quetiapine hemifumarate | 230.3 |
| Carrageenan Lambda | 216.7 |
| PEG 8000 | 92.0 |
| Fumaric acid | 47.0 |
| Povidone K30 | 28.0 |
| Mg stearate | 6.00 |

### Example 26: Hot melt granulation

| Ingridient | mg/tablet core |
|---|---|
| Kvetiapin hemifumarat | 230.3 |
| Carrageenan Lambda | 193.7 |
| PEG 4000 | 83.02 |
| Fumaric acid | 90.0 |
| Mg stearate | 3.00 |

PEG 4000 is melted and all other ingredients except Mg stearate are added and mixed. Afterwards the mixture is cooled and passed through the sieve. To the obtained mixture Mg stearate is added, homogenous mixture is finally mixed and compressed on tabletting machine.

## Claims

1. A sustained-release pharmaceutical composition comprising quetiapine or its salt and lambda-carrageenan, wherein the composition is present in the form of a tablet.

2. Pharmaceutical composition according to claim 1, wherein quetiapine is in the form of quetiapine hemifumarate.

3. Pharmaceutical composition according to any one of the preceding claims, further comprising an organic acid.

4. Pharmaceutical composition according to claim 3, wherein organic acid is selected from the group consisting of citric acid, fumaric acid, tartaric acid, benzoic acid, malic acid, ascorbic acid, succinic acid and mixtures thereof.

5. Pharmaceutical composition according to any one of the preceding claims, further comprising an anionic polymer.

6. Pharmaceutical composition according to claim 5, wherein the anionic polymer is selected from the group consisting of cellulose acetate phthalate, methacrylic acid copolymer, cellulose acetate succinate, polyvinyl acetate phthalate, hydroxypropylmethyl cellulose phthalate and/or mixtures thereof.

7. Pharmaceutical composition according to any one of the preceding claims, further comprising polyethylene glycol.

8. Pharmaceutical composition according to claim 7, wherein the polyethylene glycol having a molecular weight within the range from 4,000 to 8,000.

9. Pharmaceutical composition according to any one of the preceding claims, further comprising mannitol.

10. Pharmaceutical composition according to any one of the preceding claims comprising:
25 to 55 wt.-%, preferably 30 to 45 wt.-% of quetiapine, preferably in the form of quetiapine hemifumarate,
15 to 65 wt.-%, preferably 20 to 55 wt.-% of non-gelling carrageenan, preferably lambda-carrageenan,
and optionally 1 to 2 wt.-% of lubricant, preferably magnesium stearate,
based on the total weight of the composition.

11. Pharmaceutical composition according to claim 10, additionally comprising:
10 to 40 wt.-% diluent, preferably, lactose, in particular alpha-lactose monohydrate, mannitol and/or diclacium phosphate dihydrate, and/or
5 to 15 wt.-% organic acid, preferably citric acid and/or fumaric acid, and/or
10 to 35 wt.-% anionic polymer, in particular HPMC acetate succinate and/or methacrylic acid copolymer, and/or
12 to 24 wt.-% polyethylene glycol, in particular polyethylene glycol having an average molecular weight of 4,000,
based on the total weight of the composition.

12. Pharmaceutical composition according to any one of the preceding claims wherein the quetiapine is in the form of quetiapine hemifumarate of crystalline form I of WO 03/080065 .

13. Pharmaceutical composition according to any one of the preceding claims comprising 50 to 800 mg of quetiapine in the form of quetiapine hemifumarate.

## Patentansprüche

1. Pharmazeutische Depot-Zusammensetzung (Sustained-Release), die Quetiapin oder dessen Salz und Lambda-Carrageen enthält, wobei die Zusammensetzung in Form einer Tablette vorliegt.

2. Pharmazeutische Zusammensetzung nach Anspruch 1, bei der das Quetiapin in Form von Quetiapinhemifumarat vorliegt.

3. Pharmazeutische Zusammensetzung nach einem der vorhergehenden Ansprüche, die ferner organische Säure enthält.

4. Pharmazeutische Zusammensetzung nach Anspruch 4, bei der die organische Säure aus der Gruppe bestehend aus Citronensäure, Fumarsäure, Weinsäure, Benzoesäure, Äpfelsäure, Ascorbinsäure, Bernsteinsäure und Mischungen davon ausgewählt ist.

5. Pharmazeutische Zusammensetzung nach einem der vorhergehenden Ansprüche, die ferner anionisches Polymer enthält.

6. Pharmazeutische Zusammensetzung nach Anspruch 6, bei der das anionische Polymer aus der Gruppe bestehend aus Celluloseacetatphthalat, Methacrylsäurecopolymer, Celluloseacetatsuccinat, Polyvinylacetatphthalat, Hydroxypropylmethylcellulosephthalat und/oder Mischungen davon ausgewählt ist.

7. Pharmazeutische Zusammensetzung nach einem der vorhergehenden Ansprüche, die ferner Polyethylenglycol enthält.

8. Pharmazeutische Zusammensetzung nach Anspruch 8, bei der das Polyethylenglycol ein Molekulargewicht im Bereich von 4000 bis 8000 aufweist.

9. Pharmazeutische Zusammensetzung nach einem der vorhergehenden Ansprüche, die ferner Mannitol enthält.

10. Pharmazeutische Zusammensetzung nach einem der vorhergehenden Ansprüche, die
25 bis 55 Gew.-%, vorzugsweise 30 bis 45 Gew.-% Quetiapin, vorzugsweise in Form von Quetiapinhemifumarat,
15 bis 65 Gew.-%, vorzugsweise 20 bis 55 Gew.-% nicht gelbildendes Carrageen, vorzugsweise Lambda-Carrageen,
und wahlweise 1 bis 2 Gew.-% Schmierstoff, vorzugsweise Magnesiumstearat, enthält,
bezogen auf das Gesamtgewicht der Zusammensetzung.

11. Pharmazeutische Zusammensetzung nach Anspruch 10, die zusätzlich
10 bis 40 Gew.-% Streckmittel, vorzugsweise Lactose, insbesondere α-Lactosemonohydrat, Mannitol und/oder Dicalciumphosphatdihydrat, und/oder
5 bis 15 Gew.-% organische Säure, vorzugsweise Citronensäure und/oder Fumarsäure, und/oder
10 bis 35 Gew.-% anionisches Polymer, insbesondere HPMC-Acetatsuccinat und/oder Methacrylsäurecopolymer, und/oder 12 bis 24 Gew.-% Polyethylenglycol, insbesondere Polyethylenglycol mit einem durchschnittlichen Molekulargewicht von 4000, enthält,
bezogen auf das Gesamtgewicht der Zusammensetzung.

12. Pharmazeutische Zusammensetzung nach einem der vorhergehenden Ansprüche, bei der das Quetiapin in Form von Quetiapinhemifumarat der Kristallform I der WO 03/080065 vorliegt.

13. Pharmazeutische Zusammensetzung nach einem der vorhergehenden Ansprüche, die 50 bis 800 mg Quetiapin in Form von Quetiapinhemifumarat enthält.

## Revendications

1. Composition pharmaceutique à libération prolongée, comprenant de la quétiapine, ou de l'un de ses sels, et du lambda-carraghénane, laquelle composition se présente sous forme de comprimé.

2. Composition pharmaceutique conforme à la revendication 1, dans laquelle la quétiapine se trouve à l'état d'hémifumarate de quétiapine.

3. Composition pharmaceutique conforme à l'une des revendications précédentes, qui comprend en outre un acide organique.

4. Composition pharmaceutique conforme à la revendication 3, dans laquelle l'acide organique est choisi dans l'ensemble constitué par les suivants : acide citrique, acide fumarique, acide tartrique, acide benzoïque, acide malique, acide ascorbique et acide succinique, ainsi que leurs mélanges.

5. Composition pharmaceutique conforme à l'une des revendications précédentes, qui comprend en outre un polymère anionique.

6. Composition pharmaceutique conforme à la revendication 5, dans laquelle le polymère anionique est choisi dans l'ensemble constitué par les suivants : un acétate-phtalate de cellulose, un copolymère d'acide méthacrylique, un acétate-succinate de cellulose, un poly(acétate-phtalate de vinyle), un phtalate d'hydroxypropyl-méthyl-cellulose, et/ou les mélanges de tels composés.

7. Composition pharmaceutique conforme à l'une des revendications précédentes, qui comprend en outre un polyéthylène-glycol.

8. Composition pharmaceutique conforme à la revendication 7, dans laquelle le polyéthylène-glycol présente une masse molaire située dans l'intervalle allant de 4000 à 8000.

9. Composition pharmaceutique conforme à l'une des revendications précédentes, qui comprend en outre du mannitol.

10. Composition pharmaceutique conforme à l'une des revendications précédentes, qui comprend :
- de 25 à 55 % en poids, et de préférence de 30 à 45 % en poids, de quétiapine, de préférence à l'état d'hémifumarate de quétiapine,
- de 15 à 65 % en poids, et de préférence de 20 à 55 % en poids, d'un carraghénane non-gélifiant, qui est de préférence du lambda-carraghénane,
- et en option, de 1 à 2 % en poids d'un lubrifiant, qui est de préférence du stéarate de magnésium,
par rapport au poids total de la composition.

11. Composition pharmaceutique conforme à la revendication 10, qui comprend en outre :
- de 10 à 40 % en poids d'un diluant, qui est de préférence du lactose, en particulier du monohydrate d'alpha-lactose, du mannitol et/ou du dihydrate de phosphate dicalcique,
- et/ou de 5 à 15 % en poids d'un acide organique, qui est de préférence de l'acide citrique et/ou de l'acide fumarique,
- et/ou de 10 à 35 % en poids d'un polymère anionique, qui est en particulier de l'acétate-succinate d'hydroxypropyl-méthyl-cellulose et/ou un copolymère d'acide méthacrylique,
- et/ou de 12 à 24 % en poids d'un polyéthylène-glycol, qui est en particulier un polyéthylène-glycol dont la masse molaire moyenne vaut 4000,
par rapport au poids total de la composition.

12. Composition pharmaceutique conforme à l'une des revendications précédentes, dans laquelle la quétiapine se trouve à l'état d'hémifumarate de quétiapine sous la forme cristalline 1 décrite dans le document WO 03/080065.

13. Composition pharmaceutique conforme à l'une des revendications précédentes, qui comprend de 50 à 800 mg de quétiapine à l'état d'hémifumarate de quétiapine.
